# EUROPEAN PATENT APPLICATION

(11) **EP 1 293 176 A2**
(43) Date of publication of application: **19.03.2003**
(21) Application number: 02256353.0
(22) Date of filing: 13.09.2002
(51) Int. Cl.: A61F 2/01, A61F 2/06

(54) **A percutaneous system for opening a stenosed vessel**

(30) Priority: 14.09.2001 US 952375
(71) Applicant: Cordis Corporation, Miami Lakes Florida 33014 (US)
(72) Inventor: Fischell, David R., Fair Haven, NJ 07704 (US); Fischell, Tim A., Richland, MI 49083 (US)
(74) Representative: Belcher, Simon James

(57) **Abstract**

A percutaneous system for opening a stenosed vessel of a human body comprises a catheter having a distal end and a proximal end. The distal end of the catheter comprises a vessel opening subsystem adapted to open the stenosed vessel, and a distal protection subsystem adapted to be deployed before use of the vessel opening subsystem. The distal protection subsystem provides a means to trap distal embolic material released during the vessel revascularisation performed using the opening subsystem.

## Description

This invention is in the field of percutaneous devices that are used to open a vessel of the human body. The device can have integrated distal emboli protection.

In balloon angioplasty of vessels of the human body, embolic debris can be released and wash down stream. Existing distal emboli protection devices such as that sold by Cordis Corporation under the trade mark Angiogard are integrated into a guide wire. This device is delivered to the site of the obstruction before the device being used for revascularisation of the vessel. It would be of significant advantage to be able to deliver the revascularisation device over a conventional guidewire and have the distal emboli protection integrated into the revascularisation device itself.

The present invention is the incorporation of a distal emboli collection device into the distal section of a percutaneously inserted device for reopening an obstruction in a vessel of the human body. Such reopening or revascularisation devices include balloon angioplasty catheters, atherectomy catheters and stents. Stents used for recannalisation include balloon expandable stents such as the stents available from Cordis Corporation under the trade marks Palmaz, Corinthian and BX Velocity, and self-expanding stents such as those available from Cordis Corporation under the trade marks Precise and Smart and from Boston Scientific under the trade mark Radius. As self-expanding stents often require post implant balloon dilatation, it is envisioned that one embodiment of the present invention would provide a self-expanding stent delivery system with integrated distal emboli protection and a built in angioplasty balloon for post dilatation.

Thus, it is an object of the present invention to provide the capability for both distal emboli protection and vessel opening.

Another object of the present invention to have a balloon angioplasty catheter with integrated distal emboli protection.

Still another object of the present invention is to have a balloon expandable stent delivery system with integrated distal emboli protection.

Still another object of the present invention is to have a self-expanding stent delivery system with integrated distal emboli protection.

Yet another object of the present invention is to have a self-expanding stent delivery system with integrated distal emboli protection and a built-in angioplasty balloon.

Embodiments of the invention will now be described by way of example with reference to the accompanying drawings, in which:
FIG. 1 is a longitudinal cross section of the distal end of the present invention system that includes a balloon to be used for expansion of a balloon expandable stent or post dilatation of a self expanding stent.
FIG. 2 is a longitudinal cross section of the distal end of an alternate embodiment of the present invention system for delivery of self-expanding stents that does not include a balloon for post dilatation of a self-expanding stent.
FIG. 3 is a longitudinal cross section of a balloon dilatation catheter with integrated distal protection.
FIG 4A is a longitudinal cross section of the system of FIG. 1 after it has been positioned at the site of a stenosis in a vessel of a human body.
FIG. 4B is a longitudinal cross section of the system of FIG. 1 after the sheath has been pulled back enough to deploy the distal protection subsystem.
FIG. 4C is a longitudinal cross section of the system of FIG. 1 after the sheath has been pulled back completely and the stent has been deployed
FIG. 4D is a longitudinal cross section of the system of FIG. 1 after the sheath has been advanced until it has partly collapsed the distal protection sub system.
FIG. 4E is a longitudinal cross section of the system of FIG. 1 after the distal protection subsystem has been completely collapsed but before the system is withdrawn from the human body.

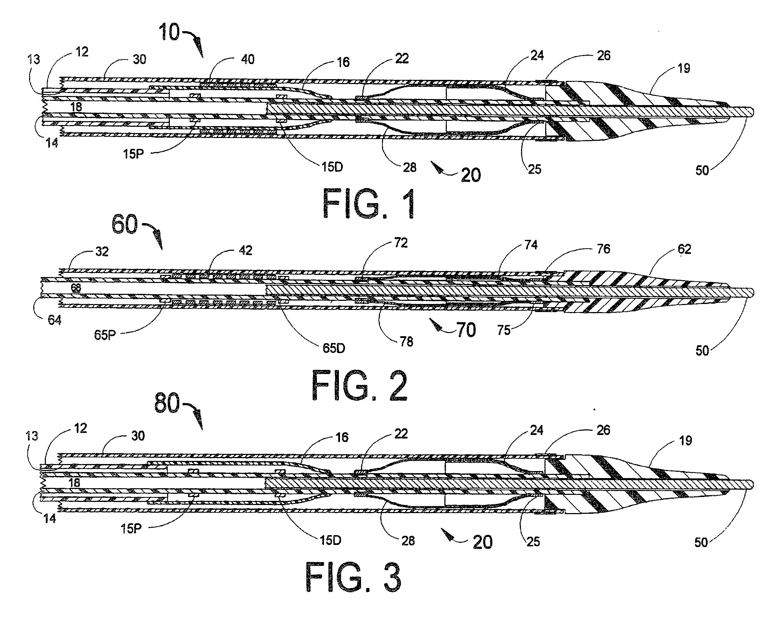

Referring to the drawings, FIG. 1 is a longitudinal cross section of the distal end of the present invention system 10 that includes a balloon 16 to be used for expansion of the stent 40. The stent 40 can be either a balloon expandable stent or a self-expanding stent. If the stent 40 is a balloon expandable stent, then the balloon 16 is used for both initial deployment and post-dilatation. If the stent 40 is a self-expanding stent then the balloon 40 is used only for post-dilatation. The system 10 is designed to be advanced over a guide wire 50 and may be either an over-the-wire type system where the guidewire lumen 18 runs the entire length of the system 10 of a rapid exchange or monorail type system where the proximal exit of the guidewire lumen 18 is between the proximal end of the system 10 and the proximal end of the balloon 16. The balloon inflation lumen 13 located between the inner shaft 12 and the outer shaft 14 provides fluid communication between a balloon inflation device (not shown) attachable to the proximal end of the system 10 and the inside of the balloon 16. The technology for the design of angioplasty balloon catheters and stent delivery balloon catheters are well known.

Distal and proximal marker bands 15D and 15P respectively are placed on the inner shaft 12 to provide a radiopaque maker for the ends of the stent 40. A radiopaque marker band 26 is attached at the distal end of the sheath 30 to provide an indication of sheath position. The sheath 30 is shown in FIG. 1, as it would be during advancement of the system 10 into the human body. In this position, the distal end of the sheath 30 is advanced as far as possible in the distal direction until it nests into the proximal end of the flexible distal tip 19 of the system 10. The distal protection subsystem 20 is positioned coaxially around the inner shaft 12 between the distal end of the balloon 16 and the proximal end of the flexible distal tip 19.

The mechanical structure of the distal protection subsystem 20 comprises 3 or more spring spokes 28 that are fixedly attached to a proximal radiopaque ring 22 at one end and a distal radiopaque ring 25 at the other end. Typically, the proximal radiopaque ring 22 is attached to the inner shaft 12 and the distal radiopaque ring 25 is adapted to move slidably over the inner shaft 12.

FIG. 1 shows the distal protection subsystem 20 spring spokes 28 in their folded position constrained by the sheath 30. When the sheath 30 is pulled in a proximal direction past the proximal ring 22, the spring spokes 28 will move outward causing the distal ring 25 to slide in a proximal direction along the inner shaft 12. The spring spokes 28 would typically be made from a superelastic metal such as Nitinol with a memory position of maximum opening set at a diameter larger than the vessel size in which the device would be used.

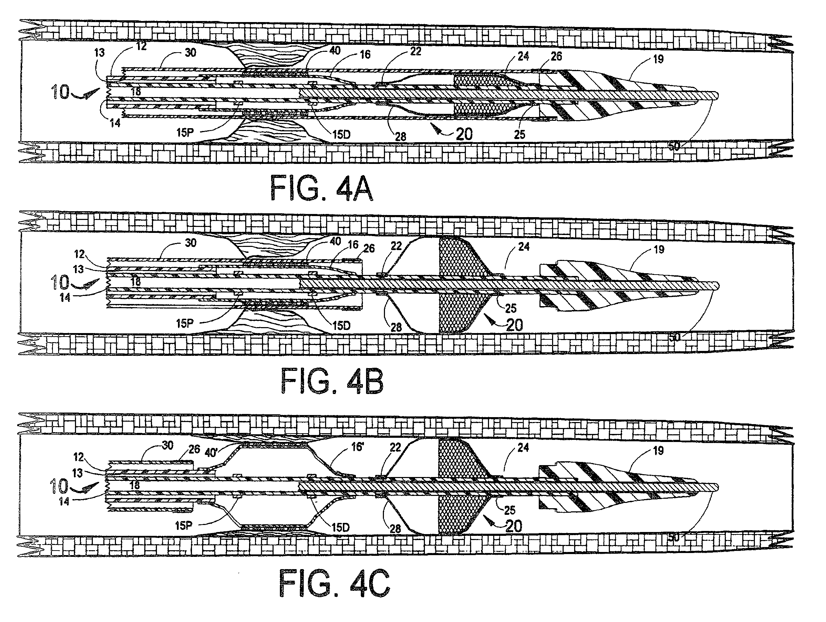

An expandable filter 24 is attached to the distal portion of the spring spokes 28 so that as the spokes 28 expand outward to the wall of a blood vessel, the filter 24 is deployed. Such expandable filters are well known in the art. The spacing of the rings 22 and 25 provide a visible indication of the state of openness of the filter 24 of the distal protection subsystem 20. Operation of the invention of FIG. 1 is described in association with FIGs. 4A through 4E that follows.

FIG. 2 is a longitudinal cross section of the distal end of an alternate embodiment of the present invention system 60 for delivery of self-expanding stents that does not include a balloon for post dilatation of a self-expanding stent 42. The system 60 comprises a shaft 64 with guidewire lumen 68, proximal and distal radiopaque marker bands 65P and 65D, a sheath 32 with distal radiopaque marker 76, a flexible distal tip 62, a guidewire 50 and a distal protection subsystem 70 having proximal radiopaque ring 72, distal radiopaque ring 75, spring spokes 78 and expandable filter 74. The system 60 is used for treatment of a stenosis of a vessel of the human body as follows:
1. The system 60 is advanced through the body over the guidewire 50 until the stent 42 as marked by the markers 65P and 65D is positioned at the site of a stenosis in a vessel such as a carotid artery.
2. The sheath 32 is then retracted to first allow expansion of the distal protection subsystem 70 and then the self-expanding stent 42.
3. The sheath 32 is advanced until the distal end of the sheath 32 collapses the spring spokes 78 thus retracting the distal protection subsystem 70.
4. The system 60 is removed from the body.

It is envisaged that the self-expanding stent 42 could be made from spring steel or a memory metal such as Nitinol. The proximal exit for the guidewire lumen 68 can be at the proximal end of the system 60 (an over-the-wire system) or between the proximal end and the proximal marker 65P (a rapid exchange system). The function of the distal protection subsystem 70 of FIG. 2 is essentially the same as the subsystem 20 of FIG. 1, the difference being that the system 10 of FIG. 1 provides an integrated balloon 16 that is needed to deploy a balloon expandable stent or to post dilate a self expanding stent.

It is also envisaged that an angioplasty balloon could be fixedly attached to the outside of the sheath 32 to allow for post dilatation of the self-expanding stent 42.

FIG. 3 is a longitudinal cross section of the distal end of another embodiment of the present invention system 80 that uses an angioplasty balloon 86 to open a stenosis of a vessel of the human body or for post dilatation of a balloon expandable or self expanding stent. The system 80 is designed to be advanced over a guide wire 50 and may be either an over-the-wire type system where the guidewire lumen 18 runs the entire length of the system 80 of a rapid exchange or monorail type system where the proximal exit of the guidewire lumen 18 is between the proximal end of the system 80 and the proximal end of the balloon 16.

The balloon inflation lumen 13 located between the inner shaft 12 and the outer shaft 14 provides fluid communication between a balloon inflation device (not shown) attachable to the proximal end of the system 80 and the inside of the balloon 86. The technology for the design of angioplasty balloon catheters is well known. Distal and proximal marker bands 15D and 15P respectively are placed on the inner shaft 12 to provide a radiopaque maker for the ends of the balloon 86. A radiopaque marker band 26 is attached at the distal end of the sheath 30 to provide an indication of sheath position.

The sheath 30 is shown in FIG. 1, as it would be during advancement of the system 80 into the human body. In this position, the distal end of the sheath 30 is advanced as far as possible in the distal direction until it nests into the proximal end of the flexible distal tip 19 of the system 10. The distal protection subsystem 20 is positioned coaxially around the inner shaft 12 between the distal end of the balloon 40 and the proximal end of the flexible distal tip 19.

The mechanical structure of the distal protection subsystem 20 comprises three or more spring spokes 28 that are fixedly attached to a proximal radiopaque ring 22 at one end and a distal radiopaque ring 25 at the other end. Typically, the proximal radiopaque ring 22 is attached to the inner shaft 12 and the distal radiopaque ring 25 is adapted to move slidably over the inner shaft 12. FIG. 1 shows the spring spokes 28 in their folded position constrained by the sheath 30. When the sheath 30 is pulled in a proximal direction past the proximal ring 22, the spring spokes 28 will move outward causing the distal ring 25 to slide in a proximal direction along the inner shaft 12. The spring spokes 28 would typically be made from a superelastic metal such as Nitinol with a memory position of maximum opening set at a diameter larger than the vessel size in which the device would be used.

An expandable filter 24 is attached to the distal portion of the spring spokes 28 so that as the spokes 28 expand outward to the wall of a blood vessel, the filter 24 is deployed. Such expandable filters are well known in the art. The spacing of the rings 22 and 25 provide a visible indication of the state of openness of the filter 24 of the distal protection subsystem 20. Operation of the invention of FIG. 1 is as follows:
1. The system 80 is advanced over the guidewire 50 until the balloon 86 is positioned at the stenosis of the vessel through imaging of the proximal and distal radiopaque markers 15P and 15D respectively.
2. The sheath 30 is then pulled back until the distal protection subsystem 20 is fully opened against the vessel wall.
3. The sheath is then pulled back until the balloon 16 is completely uncovered.
4. The balloon 86 is then expanded as in a typical angioplasty procedure.
5. The balloon 86 is deflated.
6. The sheath 30 is advanced until the distal end marker 24 is once more positioned at the distal end of the flexible distal tip 19. This will cause retraction of the spring spokes 28 of the distal protection subsystem 20.
7. The system 80 is then removed from the body.

It is envisaged that the system 80 could be used not only for opening a stenosis, but for post dilatation of a deployed stent.

FIG 4A is a longitudinal cross section of the system 10 of FIG. 1 after it has been positioned at the site of a stenosis in a vessel of a human body.

FIG. 4B is a longitudinal cross section of the system 10 of FIG. 1 after the sheath 30 has been pulled back enough to deploy the distal protection subsystem 20. This configuration completely filters the blood distal to the stent 40 through the filter 24 and should catch any distal emboli that result from stent deployment or balloon dilatation.

FIG. 4C is a longitudinal cross section of the system 10 of FIG. 1 after the sheath 30 has been pulled back completely, the stent 40 has been deployed, and the balloon 16 has been expanded inside of the stent 40.

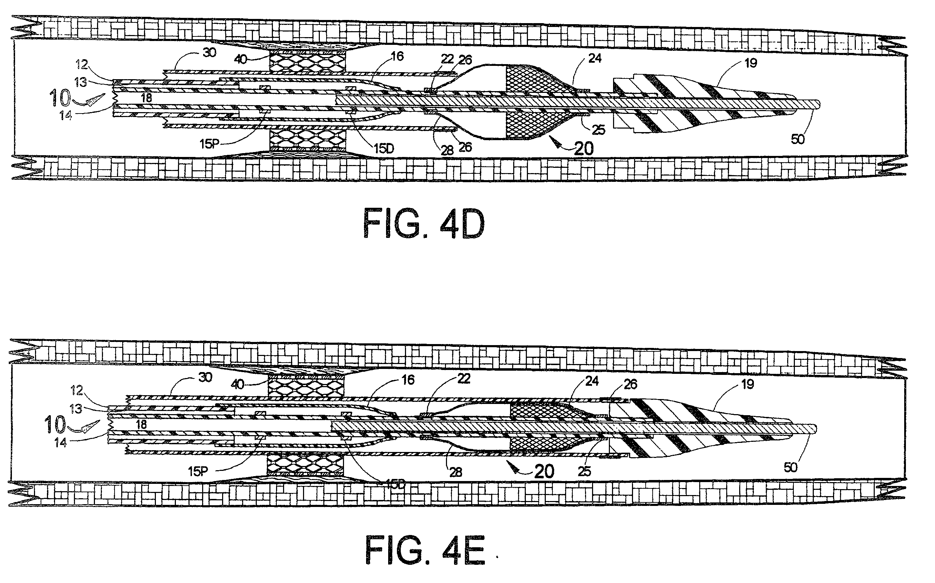

FIG. 4D is a longitudinal cross section of the system 10 of FIG. 1 after the balloon 16 has been deflated and the sheath 30 has been advanced until it has partly collapsed the spring spokes 28 of the distal protection subsystem 20.

FIG. 4E is a longitudinal cross section of the system 10 of FIG. 1 after the distal protection subsystem 20 has been completely collapsed but before the system 10 is withdrawn from the human body.

In all of the embodiments of the present invention as shown in FIGs. 1 through 4E inclusive, each system includes both a distal protection subsystem and a vessel opening subsystem. The vessel opening subsystems are specifically, the stents 40 and 42 of FIGs. 1 and 2 and the angioplasty balloon of FIG. 3.

## Claims

1. A percutaneous system for opening a stenosed vessel of a human body comprising a catheter having a distal end and a proximal end, the distal end of the catheter comprising a vessel opening subsystem adapted to open the stenosed vessel and a distal protection subsystem adapted to be deployed before use of the vessel opening subsystem, the distal protection subsystem providing a means to trap distal embolic material released during the vessel revascularisation performed using the opening subsystem.

2. The system of claim 1 wherein the vessel opening subsystem is a balloon expandable stent.

3. The system of claim 1 wherein the vessel opening subsystem is a self-expanding stent.

4. The system of claim 1 wherein the vessel opening subsystem is a balloon angioplasty catheter.

5. The system of claim 1 further comprising a sheath that when moved in the proximal direction deploys the distal protection subsystem and when pushed in the distal direction is adapted to retract the distal protection subsystem.

6. The system of claim 3 further comprising a post-dilatation balloon located coaxially under the self-expanding stent.
